(19) Europäisches Patentamt
European Patent Office
Office européen des brevets

(11) Numéro de publication: **0 036 372**
**B1**

(12) # FASCICULE DE BREVET EUROPEEN

(45) Date de publication du fascicule du brevet: **18.07.84**

(21) Numéro de dépôt: **81400405.7**

(22) Date de dépôt: **17.03.81**

(51) Int. Cl.³: **A 61 K 35/30,**
A 61 K 39/08,
A 61 K 39/106,
A 61 K 35/74

(54) **Nouveaux dérivés de gangliosides, leur préparation et leur application.**

(30) Priorité: **17.03.80 FR 8005891**

(43) Date de publication de la demande:
**23.09.81 Bulletin 81/38**

(45) Mention de la délivrance du brevet:
**18.07.84 Bulletin 84/29**

(84) Etats contractants désignés:
**AT BE CH DE FR GB IT LI LU NL SE**

(56) Documents cités:
**EP - A - 0 016 702**
**FR - A - 2 403 081**
**FR - A - 2 403 098**
**FR - A - 2 422 699**
**US - A - 4 125 492**

**CHEMICAL ABSTRACTS, vol. 78, no. 23, 11 juin 1973, page 53, abrégé 144127e Columbus, Ohio, US E. HABERMANN: "Interaction of labeled tetanus toxin and toxoid with substructures of rat brain and spinal cord in vitro"**

(73) Titulaire: **INSTITUT MERIEUX Société anonyme dite:**
**17, rue Bourgelat**
**F-69002 LYON (FR)**

(72) Inventeur: **Tayot, Jean-Louis**
**1, Rue des greffières**
**F-69890 La tour de Salvagny (FR)**
Inventeur: **Tardy, Michel**
**165 Bis, rue Joliot Curie**
**F-69000 Lyon 5ème (FR)**
Inventeur: **Court, Guy**
**Les Côtes St-Pierre-La-Palud**
**F-69210 l'Arbresle (FR)**

(74) Mandataire: **Nony, Michel**
**29, rue Cambacérès**
**F-75008 Paris (FR)**

(56) Documents cités:
**CHEMICAL ABSTRACTS, vol. 89, no. 17, 23 octobre 1978, page 156, abrégé 141556k Columbus, Ohio, US B. BIZZINI: "Properties of the binding to gangliosides and synaptosomes of a fragment of the tetanus toxin molecule"**
**MED. BIOLOGY 52(4) 1974 J. HOLMGREN et al.: "Structural requirements of G11 ganglioside in toxin binding and inactivation" pages 229-233**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 036 372 B1**

Courier Press, Leamington Spa, England.

## Description

La présente invention a pour objet de nouveaux dérivés de gangliosides, leur préparation et leur application.

Le rôle des gangliosides apparaît de plus en plus important et de mieux en mieux élucidé, dans les propriétés biologiques des membranes cellulaires. Ils servent notamment de récepteurs spécifiques vis-à-vis de différentes macromolécules biologiques (hormones, virus, interféron, toxines bactériennes etc...) et leur permettent ainsi d'exprimer leurs messages respectifs, bénéfiques ou toxiques pour les cellules. Cette fonction biologique de récepteur spécifique permet de nombreuses applications dans le domaine des médicaments et dans le domaine des purifications par chromatographie d'affinité.

On sait que les gangliosides sont des glycolipides qui peuvent être isolés notamment dans le cerveau, le foie et les reins, et dont la molécule comprend, combinés chimiquement notamment des acides gras, de la sphingosine, des oses et des acides sialiques. En particulier, les gangliosides possèdent un groupement N-acyle (acyle dérivé d'un acide gras) et au moins un groupement N-acétyle d'une galactosamine.

Selon la nomenclature proposée par Svennerholm, J. Neurochem. 10,613 (1963), les divers gangliosides sont désignés par la lettre G suivie de l'une des quatre lettres M, D, T ou Q selon que le gangliosides est un mono-, di-, tri- ou tétra sialo-ganglioside. Ces lettres sont suivies d'indices numériques permettant de distinguer les gangliosides contenant une même quantité d'acide sialique mais ayant des mobilités chromatographiques différentes. Par analogie avec la nomenclature proposée par Svennerholm pour le ganglioside $G_{M1}$ on désignera ci-après par "lysogangliosides" les produits obtenus par hydrolyse totale des groupements N-acétyle et N-acyle des gangliosides en groupements $NH_2$.

Les différents gangliosides, qui sont utilisés comme produits de départ dans la présente demande, peuvent être préparés par exemple selon les méthodes déjà décrites par exemple par adsorption sur résine échangeuse d'anions suivie d'une élution sélective par un gradient de concentration croissante d'acétate de potassium dans le méthanol; voir Fredman et coll, Biochimica et Biophysica Acta (1980); voir aussi communication de Fredman et coll; "Structure and function of gangliosides", Congrès de Le Bischenberg, Strasbourg Avril 1979, édité par Plenum Press.

Si l'on parvient à fixer chimiquement un ganglioside donné à la surface d'un support chromatographique convenablement choisi, on dispose alors d'un nouveau support chromatographique doué d'une affinité spécifique vis-à-vis de la macromolécule biologique ou de la particule qui reconnaît naturellement et sélectivement ce ganglioside choisi.

Il est alors possible de purifier ou d'isoler toute une famille de macromolécules biologiques d'un grand intérêt thérapeutique, et d'immobiliser pour les repérer ou les isoler, les particules (ou cellules) dont certains gangliosides sont les marqueurs.

Sur un support recouvert de ganglioside $G_{D1a}$, $G_{D1b}$ ou $G_{T1}$ il devient possible de fixer la toxine tétanique et de la purifier pour préparer ensuite un vaccin antitétanique plus purifié et peut-être encore plus immunogène que le vaccin actuel.

Sur un support recouvert de $G_{M2}$ il devient possible d'isoler et de purifier l'interféron qui suscite actuellement beaucoup d'espoirs en thérapeutique anti-virale et anti-tumorale.

Depuis quelques années, de nombreux supports chromatographiques et de nombreuses réactions chimiques ont été développés pour réaliser la fixation d'un ligand (molécule ou récepteur doué de propriétés de reconnaissance spécifique) sur un support notamment, en vue d'une utilisation en chromatographie d'affinité.

Les fonctions chimiques généralement nécessaires sur le support sont des fonctions alcool-OH, amine-$NH_2$, acide carboxylique-COOH,

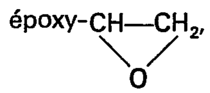

aldéhyde-CH=O.

Les fonctions chimiques complémentaires sur le ligand sont choisies parmi les mêmes fonctions, selon les principes connus. Ces principes sont décrits par exemple dans l'ouvrage "Affinity Chromatography, principles and methods", Pharmacia Fine Chemicals, Upsalla, Suède (1974).

Dans le cas particulier des gangliosides et des glycolipides, deux types de fonctions chimiques réactives sont naturellement disponibles: il y a en effet de nombreuses fonctions alcool et souvent un ou plusieurs acides carboxyliques portés par l'acide N-acétyl neuraminique ou d'autres acides sialiques. Cependant l'expérience a montré qu'une fixation par l'intermédiaire de ces fonctions détruisait ou rendait inutilisables les propriétés d'affinité spécifique attendues d'un tel support.

Le problème est donc de faire apparaître d'autres fonctions chimiques sur ces gangliosides ou glycolipides sans détruire leur activité biologique.

Plusieurs méthodes développées par Uemura et al (1976) et Wiegandt et al (1970) consistent à oxyder la portion terminale de la sphingosine au niveau de la double liaison pour faire apparaître une fonction réactive de type époxy ou aldéhyde.

Ces méthodes n'ont cependant donné que des résultats peu exploitables.

Une autre méthode, développée en 1963 par Taketomi et al, J of Biochem. 54,5,444 (1963), consiste à transformer les gangliosides en lysogangliosides. Les fonctions N-acétyl et N-acyl sont totalement hydrolysées en fonctions aminées —$NH_2$ par chauffage à 120°C à reflux pendant 2 heures dans un mélange de butanol-potasse 10 N (90:10). Après extraction dans une phase aqueuse, et concentration par évaporation, le lysoganglioside ainsi obtenu peut être facilement couplé sur différentes matrices. C'est ainsi que du lysoganglioside $G_{M1}$ peut être couplé à différents supports Polysaccharidiques pour purifier la toxine cholérique (voir demande de brevet français 77 28163).

Cependant en voulant appliquer la même méthode pour fixer les gangliosides $G_{D1a}$, $G_{D1b}$ ou $G_{T1}$ récepteurs de la toxine tétanique, il n'a pas été possible de réaliser un support doué d'affinité pour la toxine tétanique, contrairement à ce qui était normalement attendu.

Il a maintenant été découvert qu'il est possible de transformer les gangliosides par hydrolyse ménagée, en de nouveaux dérivés de ganglioside "activés" qui ne présentent pas ces inconvénients. Ces nouveaux dérivés possèdant des groupements réactifs permettant de les fixer sur différents supports, de diverses manières, sans perte de leurs propriétés de ligands doués d'affinité spécifique vis-à-vis de macromolécules biologiques.

Cette méthode est applicable, plus généralement à tous les gangliosides possédant plusieurs groupements N-acyle. Il convient de préciser que, par convention, dans la présente demande, l'expression "ganglioside" englobe ces divers glycolipides.

L'utilisation de ces supports en chromatographie d'affinité a permis, dans le cas du dérivé du ganglioside $G_{D1b}$ ou $G_{T1}$ de purifier la toxine tétanique en une seule étape, montrant que les conditions nouvelles d'activation chimique des gangliosides respectaient leur propriétés biologiques. Une purification de cette nature, aussi simple et aussi rapide, n'avait jamais été décrite à notre connaissance.

Ce procédé d'activation ménagée des gangliosides a également été appliqué avec succès au ganglioside $G_{M1}$ et à divers autres gangliosides purs ou en mélanges.

La présente invention a pour objet de nouveaux dérivés de gangliosides, caractérisés par le fait qu'ils sont des produits de désacylation partielle des gangliosides, qu'ils présentent des groupements amine libre pouvant être mis en évidence par une réaction positive dans le test à la ninhydrine, qu'ils sont mobiles en chromatographie sur couche mince de gel de silice dans le système chloroforme-méthanol-eau (60:32:7), et qu'ils présentent les propriétés d'affinité spécifique des gangliosides dont ils dérivent, lesdits dérivés pouvant être couplés à des supports solides, par l'intermédiaire des groupements amine apparus lors de la désacylation partielle, sans perte desdites propriétés d'affinité spécifique.

L'invention a également pour objet un procédé de préparation des nouveaux dérivés activés de gangliosides.

Selon ce procédé pour activer chimiquement les gangliosides tout en conservant l'essentiel de leurs propriétés biologiques, il suffit de traiter ceux-ci avec une solution aqueuse basique à une température comprise entre 0 et 120°C, ladite température étant d'autant plus basse que le milieu est plus basique. Pour une solution finale d'alcalinité comparable à la potasse ou la soude normale, il importe de ne pas dépasser 100°C. Pour une alcalinité comparable à la potasse ou la soude décinormale, il importe de ne pas dépasser 120°C. Le plus souvent on choisit les conditions qui permettent d'opérer entre 40 et 100°C.

Le temps de réaction doit être suffisant pour qu'apparaissent des groupements amine libre, mais il faut en outre arrêter la réaction avant désacylation totale. Le temps de réaction peut être déterminé facilement dans chaque cas par une expérimentation simple, en utilisant par exemple le test à la ninhydrine, ou le text de Sanger au dinitrofluorobenzène, en recherchant une coloration intermédiaire d'intensité inférieure à celle obtenue après désacylation complète. Généralement, le temps de réaction varie de 30 minutes à 24 heures.

Le solvant de réaction est soit l'eau, soit un mélange d'eau et de solvants organiques. Parmi les solvants organiques on citera des alcools tels que le butanol.

Parmi les bases utilisables dans le procédé de l'invention, on citera celles qui sont capables de donner dans l'eau un pH de 9 à 14, et en particulier les hydroxydes de métal alcalin comme la soude ou la potasse.

Le plus simple est de dissoudre le ganglioside choisi dans de la potasse ou de la soude normale et d'incuber cette solution à température de 60—100°C pendant 2 heures environ.

La transformation se traduit par l'apparition d'au moins une fonction —$NH_2$ résultant de l'hydrolyse partielle des fonctions N-acétyl ou N-acyl, en nombre suffisant pour permettre une fixation par des méthodes connues, au moyen de ladite fonction—$NH_2$, mais cette transformation est assez limitée pour conserver l'essentiel des propriétés biologiques des gangliosides choisis.

Il est possible de vérifier que ces conditions nouvelles d'hydrolyse basique donnent un dérivé de ganglioside très différent du lysoganglioside obtenu par hydrolyse totale dans les conditions décrites par Taketomi. En effet, en chromatographie en couche mince, sur gel de silice dans le système chloroforme-méthanol-eau (60-32-7), les lysogangliosides obtenus selon Taketomi ne migrent absolument pas,

alors que les gangliosides activés selon le présent procédé donnent un Rf égal ou inférieur à celui du ganglioside natif correspondant, mais toujours déplacé par rapport au point de départ.

Comme indiqué ci-dessus, l'invention a également pour objet les nouveaux dérivés de désacylation partielle de gangliosides couplés à des supports solides par l'intermédiaire de leurs groupements amine apparus lors de la désacylation partielle. Les gangliosides de départ sont ceux cités ci-dessus, et en particulier les gangliosides $G_{D1a}$, $G_{D1b}$, $G_{T1}$.

Pour les applications non chromatographiques notamment pour des tests biologiques, on peut également utiliser d'autres supports solides doués de propriétés adsorbantes, par exemple le polystyrène, le polypropylène, les latex, etc, ou capables de fixer un agent de couplage chimique (d'extran, cellulose, etc...) comme cela sera exposé ci-après.

Les supports solides peuvent être aussi des supports solides utilisables en chromatographie.

Ce procédé, qui comprend l'étape d'hydrolyse ménagée comme décrit précédemment, est caractérisé par le fait qu'il comprend en outre l'étape consistant à fixer sur un support solide, selon les méthodes connues, par l'intermédiaire des groupements amine apparus, le dérivé activé de ganglioside.

La réaction de couplage peut être effectuée selon les méthodes connues, comme cela a été indiqué ci-dessus en présence éventuellement d'un agent de couplage intermédiaire (dérivé bifonctionnel). Parmi les supports solides utilisables on citera les supports solides poreux. Les supports solides poreux sont par exemple des oxydes métalliques tels que la silice, l'alumine, la magnésie, etc..., ou leurs dérivés synthétiques ou naturels tels que les verres, les silicates, les borosilicates, ou le kaolin, ou des polymères organiques tels que des polysaccharides, des polymères organiques tels que des polysaccharides, des polymères vinyliques tels que des polyacryliques, des polystyrènes, etc....

Les supports minéraux poreux peuvent être des supports modifiés de façon à comporter des groupements réactifs capables de favoriser la fixation des nouveaux dérivés de gangliosides par l'intermédiaire de leurs groupements amine apparus lors de la désacylation partielle. Lesdits groupements réactifs peuvent être aussi des groupements capables de réagir avec une fonction d'un dérivé bifonctionnel (agent de couplage) dont l'autre fonction est susceptible de réagir avec lesdits groupements $NH_2$.

Les groupements réactifs capables de réagir avec les groupements $NH_2$ sont bien connus; ce sont notamment les groupements époxy, les groupements carbonylés (notamment aldéhydes), les groupements carboxyliques en présence de carbodiimides, etc....

Selon un mode de réalisation particulier, lorsque le couplage est effectué avec un support ou un agent de couplage du type époxy, le support de type époxy et les réactions d'hydrolyse ménagée et de couplage peuvent être effectuées simultanément en ajoutant le support au début ou au cours de la réaction d'hydrolyse.

Le dérivé de ganglioside peut être fixé par exemple sur des particules de polysaccharides. L'expression "polysaccharide" englobe ici les polysaccharides modifiés. Ces polysaccharides sont notamment du dextran, des celluloses, de l'amidon, de l'agarose, etc... ou encore des polysaccharides modifiés, notamment des dialkylaminoalkyl- ou di(hydroxyalkyl) aminoalkyl-polysaccharides tels que par exemple le diéthylaminoéthyl dextran, la diéthylaminoéthyl cellulose, etc....

On fixe le dérivé de ganglioside sur les polysaccharides à l'aide d'agents de couplage tels que des diépoxydes, des dicarbonylés, des épichlorhydrine ou le bromure de cyanogène, selon les méthodes connues.

Les polysaccharides peuvent être également utilisés, non pas sous forme de particules, mais sous forme de revêtement sur des particules minérales, notamment des oxydes minéraux tels que ceux mentionnés ci-dessus.

Les particules minérales poreuses revêtues de polysaccharides ou de polysaccharides modifiés peuvent être notamment celles décrites dans la demande de brevet français n° 76. 23176 ou celles décrites dans la demande de brevet français n° 77.28163.

Les particules minérales revêtues de polysaccharides, conformément à la demande de brevet français n° 76.23176 sont constituées d'un support minéral poreux, tel qu'un oxyde minéral poreux, revêtu directement sur sa surface par un polymère polysaccharidique aminé.

Le support minéral poreux peut être de la silice, de l'alumine, de la magnésie, un oxyde de titane, ou leurs dérivés synthétiques ou naturels tels que verres, borosilicates, silicates, kaolin, etc....

Le polymère polysaccharidique aminé est fixé sur le support minéral poreux par encollage.

La surface interne du support minéral poreux est par exemple inférieure ou égale à 100 $m^2/g$ et si possible comprise entre 5 et 80 $m^2/g$. Le diamètre poreux moyen est de préférence supérieur ou égal à 25 nm et si possible compris entre 50 et 1000 nm. Pour des surfaces plus grandes ou des diamètres poreux faibles la surface interne du support devient inaccessible au polymère polysaccharidique. Le support poreux minéral est par exemple de la silice ou de l'alumine et de préférence un support de silice poreuse à caractère anionique obtenue selon les procédés décrits dans les brevets français n° 1.473.239, n° 1.473.240, n° 1.475.929, n° 1.482.867, telles que les silices poreuses commercialisées par Rhone-Poulenc Chimie Fine sous les dénominations Spherosil XOB 030, XOB 015, XOB 005 et XOC 005.

Le polymère polysaccharidique aminé qui sert à imprégner et à recouvrir la surface interne du support poreux minéral doit avoir un caractère cationique prononcé et avoir de bonnes propriétés d'hydrophilie. Il doit avoir un poids moléculaire au moins égal à $10^4$ daltons et si possible être compris entre $10^5$ et $10^6$. Sa formule peut être quelconque et il peut en particulier être un dérivé aminé du dextran, de l'amidon, de la cellulose, de l'agarose ou d'un polymère naturel ou synthétique de tous les oses connus.

Les fonctions amines du polymère polysaccharidique peuvent être primaires, secondaires ou tertiaires ou éventuellement quaternaires.

Le polymère polysaccharidique aminé peut notamment correspondre à la formule:

$$R{-}(CH_2)_n{-}NR_1\ (R_2)$$

dans laquelle:

R représente un reste de polysaccharide tel que par exemple un reste de dextran, d'amidon, de cellulose ou d'agarose, n est un nombre entier de 1 à 10 et de préférence de 2 à 5, $R_1$ et $R_2$ identiques ou différents représentent un radical alkyle ou hydroxyalkyle inférieur, par exemple les radicaux suivants:

$$-CH_3,\ -CH_2,\ -CH_3,\ -CH_2OH,\ -CH_2 -CH_2OH \text{ ou } -CH_2 -CHOH-CH_3,$$

ces polymères pouvant être quaternarisés à l'aide d'un agent quaternarisant classique tel que les halogénures d'alkyle ou d'hydroxyalkyle, le sulfate de diméthyle, etc....

Parmi les polymères de ce type, on peut en particulier citer les composés connus sous les dénominations Deae Dextran (diéthylamino éthyl dextran) de poids moléculaire 500.000 et Qae Dextran (diéthylamino éthyl dextran quaternisé) vendus par la firme Pharmacia et le composé connu sous la dénomination Deae amidon (diéthylamino éthyl amidon) ainsi que les amidons cationiques tels que ceux vendus sous la dénomination commerciale Cato par la Société Roquette National.

Le polymère polysaccharidique aminé peut aussi être réticulé à l'aide d'un agent réticulant par exemple un composé de carbonylés, un diépoxyde tel que le 1—4 butanedioldiglycidyléther, ou l'épichlorhydrine ou l'épibromhydrine.

On fixe les dérivés de gangliosides sur les supports poreux ainsi revêtus, par exemple à l'aide des agents de couplage déjà mentionnés ci-dessus.

Les particules minérales poreuses revêtues de polysaccharides modifiés ou non, selon la demande de brevet français n° 77.28163 sont constituées par un support minéral poreux revêtu par un polymère polysaccharidique, ou par un polymère polysaccharidique modifié, par exemple un polysaccharide aminé tel que mentionné ci-dessus, ledit revêtement polysaccharidique étant si nécessaire stabilisé par réticulation, et sur ledit revêtement polysaccharidique ou polysaccharidique modifié se trouve greffé un dérivé de ganglioside de formule schématique $R''{-}NH_2$, R'' étant le reste de la molécule du dérivé de ganglioside, la liaison de greffage de ladite molécule répondant à la formule

$$R_3{-}CH_2{-}NH{-}R''$$

R'' étant défini comme précédemment et $R_3{-}CH_2{-}$ représentant le reste dudit polymère polysaccharidique ou polysaccharidique modifié lorsque celui-ci a été soumis à une réaction de coupure oxydante suivie d'une réduction par exemple au borohydrure.

Les supports minéraux poreux sont ceux déjà indiqués précédemment; le polymère polysaccharidique est notamment la cellulose; le polymère polysaccharidique modifié est notamment le diéthylaminoéthylcellulose; le revêtement de polymère polysaccharidique modifié ou non est si nécessaire stabilisé par réticulation, l'agent réticulant étant tel que ceux mentionnés ci-dessus.

Le procédé de préparation de ces matériaux est caractérisé par le fait que l'on effectue un revêtement du support minéral poreux par le polymère polysaccharidique ou par le polymère polysaccharidique modifié, que si désiré on transforme le revêtement polysaccharidique en revêtement polysaccharidique modifié, que l'on effectue si nécessaire une réticulation pour stabiliser le revêtement, que l'on soumet ledit revêtement polysaccharidique ou polysaccharidique modifié à une réaction de coupure oxydante selon les méthodes connues, que l'on fait réagir le produit d'oxydation ainsi obtenu avec le dérivé activé de ganglioside tel que défini ci-dessus, de formule schématique $R''{-}NH_2$, puis que l'on soumet le dérivé iminé obtenu à l'action d'un agent réducteur capable de réduire la liaison imine en liaison amine.

L'invention a également pour objet l'application des nouveaux produits de désacylation partielle de gangliosides à la purification ou à l'isolement de macromolécules, particules ou cellules biologiques, ou à leur élimination.

Cette application est caractérisée par le fait que l'on met en contact un support sur lequel est fixé le dérive activé de ganglioside ou de glycolipide tel que défini ci-dessus, avec une solution contenant ou susceptible de contenir les macromolécules ou particules ou cellules que l'on désire purifier, isoler ou éliminer. On peut ensuite soit rechercher la présence ou l'absence de la macromolécule (ou particule ou cellule) sur le support ainsi revêtu du dérivé activé (révélation selon les méthodes connues), soit séparer ladite macromolécule (ou particule ou cellule) en vue de son isolement et de sa purification. On peut ainsi fixer la toxine tétanique sur un support revêtu de dérivé activé de ganglioside $G_{D1a}$ et/ou $G_{D1b}$ et/ou $G_{T1}$.

Selon un mode de réalisation particulier, cette application est effectuée en appliquant les méthodes de la chromatographie d'affinité. Pour cela, on réalise une colonne avec un support solide sur lequel est fixé un dérivé activé de ganglioside tel que défini ci-dessus, et on fait passer dans ladite colonne une solution contenant les macromolécules que l'on désire isoler ou purifier. Si nécessaire, on élue la macromolécule à isoler lorsqu'elle a été fixée sur le dérivé de ganglioside.

Si on désire simplement éliminer une impureté par fixation de cette impureté sur le glycolipide activé, il n'est évidemment pas nécessaire d'effectuer l'élution finale.

Si par contre on désire obtenir le produit qui s'est fixé sélectivement sur le glycolipide activé, il conviendra d'effectuer l'élution finale.

Selon un mode d'exécution particulier de l'application de l'invention, on sépare la toxine tétanique d'une solution la contenant de la façon suivante:

On réalise une colonne avec un support sur lequel est fixé du ganglioside $G_{D1b}$ et/ou $G_{T1}$ activé, on fait passer sur cette colonne une solution contenant ou susceptible de contenir de la toxine tétanique, et, si désiré, on élue ensuite la toxine dans le cas où l'on désire obtenir une toxine tétanique purifiée.

Les exemples suivants illustrent l'invention:

Exemple 1—Activation du ganglioside $G_{D1b}$

10 Micromoles de $G_{D1b}$ sont dissoutes dans 1 ml de potasse N et la solution est incubée à 60°C pendant 2 heures.

La solution obtenue, qui donne un test positif à la ninhydrine, est ensuite diluée et ajustée pour le couplage du ganglioside activé selon l'une quelconque des méthodes décrites plus loin.

La même méthode est applicable aux autres gangliosides et glycolipides. On peut également activer le ganglioside $G_{D1b}$ en le faisant incuber dans la soude N à 80°C pendant 2 heures ou dans la soude N/10 à 40°C pendant 15 heures.

Exemple 2—Activation du ganglioside $G_{T1}$

10 micromoles de $G_{T1}$ sont mises en suspension dans 10 ml de mélange Butanol-Potasse 10 N (90:10); la suspension équivaut en final à de la potasse normale. Après incubation 2 heures à 60°C et addition d'un volume égal d'eau, la phase aqueuse contenant le ganglioside activé, donnant un test positif à la ninhydrine, est prélevée et ajustée pour couplage du ganglioside activé selon l'une des quelconques méthodes décrites plus loin. Lu même méthode est applicable aux autres gangliosides et glycolipides. On peut également activer le ganglioside $G_{T1}$ en le laissant incuber dans la soude N à 80°C pendant 2 heures ou dans la soude N/10 à 40°C pendant 15 heures.

Exemple 3—Activation du ganglioside $G_{D1b}$

15 micromoles de $G_{D1b}$ sont mises en suspension dans 10 ml de mélange Butanol-Potasse N (90:10). La suspension finale équivaut à de la potasse décinormale. Après incubation 2 heures à 120°C (à reflux) et addition d'un volume égal d'eau distillée, la solution aqueuse contenant le ganglioside activé est prélevée et ajustée pour couplage du ganglioside activé selon l'une quelconque des méthodes décrites plus loin. Elle présente un test positif à la ninhydrine. La même méthode est applicable aux autres gangliosides et glycolipides.

Exemple 4

Couplage des gangliosides ainsi activés sur une matrice polysaccharidique par l'intermédiaire d'un agent bifonctionnel à fonctions époxy.

Dans tous les cas cette méthode de couplage conduit à fixer le ganglioside par une liaison amine extrêmement stable dérivée de la fonction amine primaire apparue par hydrolyse ménagée, ce qui permet d'obtenir des supports de grande durée de vie.

4.1—On peut utiliser par exemple un produit dérivé de l'agarose commercialisé par Pharmacia (Uppsala-Suède) sous le nom de "epoxy activated Sepharose 6B" et réaliser la fixation selon les recommandations du fabricant. Une dose de 1 à 10 mmole/ml est recommandée. Le dosage d'acide sialique avant et après couplage permet de vérifier la bonne fixation du ligand.

4.2—On peut utiliser d'autres polysaccharides tels que la cellulose. Le couplage à l'aide de l'agent diépoxy—par exemple le 1—4 butanedioldiglycidyléther (Aldrich)—peut se faire en incubant pendant une nuit à température ambiante 10 g de cellulose dans un mélange contenant 20 ml de NaOH N, 20 ml de Butane-dioldiglycidyléther et 40 mg de $NaBH_4$.

Le lendemain le support est lavé par de l'alcool, de l'eau et enfin de la soude 0,1 N. Après essorage par filtration, le support est mis en contact pendant une nuit avec l'un des gangliosides précédemment activés et en solution dans de la soude 0,1 N. Une dose de 1 à 10 mmole/ml est recommandée. Une température d'incubation de 20 à 40°C est préférable.

4.3—Enfin, on peut utiliser également des supports minéraux poreux imprégnés de polysaccharides tels que ceux décrits antérieurement dans la demande de brevet français 76.23176 et secondairement activés par le même réactif diépoxyde. Ce support est particulièrement recommandé pour les applications industrielles.

Exemple 5

Couplage des gangliosides ainsi activés sur une matrice polysaccharidique oxydée par le periodate de sodium.

Pour cette méthode, le cellulose ou les supports poreux imprégnés de polysaccharides tels que ceux de la demande de brevet français

76.23176 sont préférables. L'agarose est en effet peu oxydé dans les mêmes conditions.

Cette méthode a déjà été appliquée (voir demande de brevet français 77.28163) pour le couplage des lysogangliosides préparés selon la méthode de Taketomi.

Les conditions pour fixer les gangliosides activés selon le présent procédé sont identiques.

10 g de support (par exemple la cellulose) sont additionnés de 100 ml de periodate de sodium 0,02 M pendant 2 heures à température ambiante.

Le support est ensuite lavé dans une solution de carbonate de sodium 0,02 M pH 9 additionnée de NaCl 10 g/l, essoré par filtration et additionné de 15 ml d'une solution à pH 9 de gangliosides activés selon l'un quelconque des exemples 1 à 5 ci-dessus. Une dose de 1 à 10 mmole/ml de support est recommandée.

Après incubation une nuit à température ambiante, du borohydrure de sodium $NaBH_4$ est ajouté pendant 2 heures à 20°C (0,2 M en final) pour réduire les liaisons imines formées en liaisons amines extrêmement stables. Là encore, les supports ainsi réalisés ont une durée de vie excellente et peuvent être réutilisés plusieurs dizaines ou centaines de fois.

Exemple 6

Couplage des gangliosides ainsi activés sur une matrice polysaccharidique par la méthode au bromure de cyanogène.

La méthode est celle décrite par Porath et al., Nature, 215,1491 (1967). En pratique, il suffit de mélanger le Sepharose activé au bromure de cyanogène, commercialisé par Pharmacia (Uppsala-Suède), à pH 11 suivant les instructions de la notice d'emploi. Une dose de 1 à 10 mmole de ganglioside activé par ml de gel est ici encore recommandée. Il est possible d'adapter cette technique à la cellulose ou aux supports minéraux poreux précédemment décrit, sans rien changer aux conditions opératoires.

Exemple 7

Application à la fixation et à la purification de la toxine tétanique.

A titre d'exemple, on décrit ci-après les résultats obtenus sur un support minéral poreux imprégné de polysaccharide suivant une méthode déjà décrite dans la demande de brevet français 76.23176. Ce support a été choisi parce qu'il présente des propriétés de résistance mécanique remarquables, le rendant très utile pour des purifications industrielles.

Le support minéral poreux est de la silice telle que celle commercialisée par Rhône-Poulenc sous le nom de Spherosil XOC 005. Sa surface spécifique est d'environ 10 m²/g, son diamètre poreux moyen est d'environ 300 nm; cette silice est imprégnée d'une couche monomoléculaire de Deae Dextran et réticulée selon le procédé déjà décrit dans la demande de brevet français 76.23176. En appliquant la méthode décrite dans l'un des exemples 6 à 8 qui précèdent, à 10 g de ce support, il est possible de fixer par exemple 50 micromoles de gangliosides $G_{D1b}$ ou $G_{T1}$, préalablement activés selon la présente invention aux exemples 1, 2, ou 3, ci-dessus. Pour cela les 10 g de support sont lavés dans une solution de soude 0,1 N et montés en colonne. Ils occupent un volume d'environ 23 ml. La colonne est équilibrée dans une solution contenant du Glycocolle (20 g/l) et du NaCl (10 g/l). Un filtrat de culture de "Clostridium tetani" contenant à l'état brut de la toxine tétanique titrant 500 unités Lf/ml est dialysé contre ce même tampon. 50 ml de cette solution impure de toxine tétanique sont ensuite filtrés sur la colonne. Toutes les impuretés ne reconnaissant pas le ganglioside $G_{D1b}$ ou $G_{T1}$ traversent la colonne. Seule la toxine tétanique se fixe sur la colonne et peut être récupérée par élution avec de l'ammoniaque 0,1 N ou un tampon pH 9 contenant du Glycocolle (20 g/l) et du NaCl (60 g/l).

Le produit récupéré contient environ 70% de l'activité biologique présente dans la culture initiale. Avec cette seule étape, la pureté de la toxine atteint et même dépasse les normes déjà publiées (voir B. Bizzini et al, Bull. Inst. Pasteur 72, 177, 1974. La toxine ainsi purifiée à une activité spécifique moyenne d'environ 3500 Lf/mg azote. Le rapport

$$\frac{\text{D.O. 280 nm}}{\text{D.O. 260 nm}}$$

est environ égal à 2. Par immunodiffusion en gélose contre un sérum anti-filtrat de culture donnant de nombreuses lignes de précipitation avec le filtrat de culture initial, on observe une seule ligne de précipitation avec la toxine tétanique ainsi purifiée. Cette ligne donne une réaction d'indentité complète avec un sérum anti-toxine spécifique de référence ou avec une toxine purifiée de référence fournie par l'Institut Pasteur.

Il convient de noter ici qu'un support préparé de la même manière avec un ganglioside $G_{D1b}$ ou $G_{T1}$, hydrolysé dans les conditions moins douces décrites par Taketomi, article cité, ne permet aucune fixation de la toxine tétanique. Quant aux mêmes gangliosides non activés, il n'est pas possible de les fixer sur un support par les méthodes décrites ci-dessus.

**Revendications**

1. Dérivés activés partiellement désacylés de gangliosides à groupement N-acyle, caractérisés par le fait lesdits gangliosides sont choisis parmi les gangliosides $G_{D1a}$, $G_{D1b}$ et $G_{T1}$, qu'ils présentent des groupements amine libres pouvant être mis en évidence par une réaction positive dans le test à la ninhydrine, qu'ils sont mobiles en chromatographie sur couche mince

de gel de silice dans le système chloroforme-méthanol-eau (60:32:7) et qu'ils présentent des propriétés d'affinité spécifique pour la toxine tétanique, lesdits dérivés pouvant être couplés à des supports solides, par l'intermédiaire des groupements amine apparus lors de la désacylation partielle, sans perte desdites propriétés d'affinité spécifique.

2. Dérivés selon la revendication 1, caractérisés par le fait que le ganglioside est le ganglioside $G_{D1a}$.

3. Dérivés selon la revendication 1, caractérisés par le fait que le ganglioside est le ganglioside $G_{D1b}$.

4. Dérivés selon la revendication 1, caractérisés par le fait que le ganglioside est le ganglioside $G_{T1}$.

5. Dérivés selon l'une quelconque des revendications 1 à 4, caractérisés par le fait que lesdits produits de désacylations partielle sont fixés sur un support solide organique ou minéral.

6. Dérivés selon la revendication précédente, caractérisés par le fait que ledit support est un polymère organique modifié ou non, ledit dérivé de ganglioside étant fixé sur le support par l'intermédiaire des groupements amine apparus lors de la désacylation partielle.

7. Dérivés selon la revendication 5, caractérisés par le fait que ledit support est un corps minéral poreux modifié pour faire apparaître des groupements réactifs capables de réagir avec les groupements réactifs d'un agent de couplage ou avec les groupements $NH_2$ desdits dérivés désacylés.

8. Procédé de préparation des dérivés selon l'une quelconque des revendications précédentes, caractérisé par le fait que l'on traite un desdits gangliosides ou un mélange desdits gangliosides avec une solution aqueuse basique à une température comprise entre 0 et 120°C, ladite température étant d'autant plus basse que le milieu est plus basique.

9. Procédé selon la revendication 8, caractérisé par le fait que l'on choisit la base de façon à opérer à température comprise entre 40 et 100°C.

10. Procédé selon l'une quelconque des revendications 8 et 9, caractérisé par le fait que le temps de réaction est choisi de façon à être inférieur au temps nécessaire pour obtenir la désacylation totale du ganglioside de départ.

11. Procédé selon la revendication 10, caractérisé par le fait que le temps de réaction est compris entre 30 minutes et 24 heures.

12. Procédé selon l'une quelconque des revendications 8 à 11, caractérisé par le fait que la base est une base capable de donner dans l'eau un pH de 9 à 14.

13. Procédé selon l'une quelconque des revendications 8 à 12, caractérisé par le fait que l'on opère avec une solution d'alcalinité comparable à la soude ou à la potasse normale, à température ne dépassant pas 100°C.

14. Procédé selon la revendication 13, caractérisé par le fait que l'on opère à 60—100°C pendant 2 heures.

15. Procédé selon l'une quelconque des revendications 8 et 10 à 12 caractérisé par le fait que l'on opère avec une solution d'alcalinité comparable à la soude décinormale, à température inférieure ou égale à 120°C.

16. Application des dérivés activés selon l'une quelconque des revendications 1 à 7, caractérisée par le fait que l'on met en contact un support, sur lequel est fixé ledit dérivé activé de ganglioside, avec une solution contenant ou susceptible de contenir la toxine tétanique que l'on désire purifier ou isoler.

17. Application selon la revendication 16, caractérisée par le fait que pour séparer la toxine tétanique d'une solution la contenant ou susceptible de la contenir, on réalise une colonne avec un dérivé activé du ganglioside $G_{D1b}$ et/ou $G_{D1a}$ et/ou $G_{T1}$ fixé sur un support, on fait passer ladite solution sur ladite colonne, et, si désiré, on élue ensuite la toxine.

**Patentansprüche**

1. Partiell desacylierte aktivierte Derivate von Gangliosiden mit Acyl-Gruppe, dadurch gekennzeichnet, daß die Ganglioside unter den Gangliosiden $G_{M1}$, $G_{M2}$, $G_{D1a}$, $G_{D1b}$ und $G_{T1}$ ausgewählt sind, daß sie freie Amingruppen aufweisen, was durch eine positive Reaktion beim Ninhydrin-Test gezeigt werden kann, daß sie bei der Kieselgel-Dünnschichtchromatographie im System Chloroform-Methanol-Wasser (60:32:7) beweglich sind und daß sie die spezifischen Affinitätseigenschaften gegenüber Tetanustoxin aufweisen, wobei diese Derivate an feste Träger durch die bei der partiellen Desacylierung auftretenden Amingruppen ohne Verlust der spezifischen Affinitätseigenschaften gekuppelt sind.

2. Derivate nach Anspruch 1, dadurch gekennzeichnet, daß das Gangliosid das Gangliosid $G_{D1a}$ ist.

3. Derivate nach Anspruch 1, dadurch gekennzeichnet, daß das Gangliosid das Gangliosid $G_{D1b}$ ist.

4. Derivate nach Anspruch 1, dadurch gekennzeichnet, daß das Gangliosid das Gangliosid $G_{T1}$ ist.

5. Derivate nach einem der Ansprüche 1 bis 4, dadurch gekennzeichnet, daß die Produkte der partiellen Desacylierung auf einem festen organischen oder mineralischen Träger fixiert ist.

6. Derivate nach dem vorhergehenden Anspruch, dadurch gekennzeichnet, daß der Träger ein modifiziertes oder nicht-modifiziertes organisches Polymer ist, wobei das Gangliosid-Derivat auf dem Träger durch bei der partiellen Desacylierung aufgetretene Amingruppen fixiert ist.

7. Derivate nach Anspruch 5, dadurch gekennzeichnet, daß der Träger ein mineralischer, poröser Körper ist, modifiziert,

um reaktive Gruppen auftreten zu lassen, die mit den reaktiven Gruppen eines Kupplungsmittels oder mit den $NH_2$-Gruppen der desacylierten Derivate zu reagieren vermögen.

8. Verfahren zur Herstellung von Derivaten gemäß einem der vorhergehenden Ansprüche, dadurch gekennzeichnet, daß man eines der Ganglioside oder ein Gemisch der Ganglioside mit einer wässrig-basischen Lösung bei einer Temperatur zwischen 0 und 120°C behandelt, wobei die Temperatur umso tiefer ist, je basischer das Medium ist.

9. Verfahren nach Anspruch 8, dadurch gekennzeichnet, daß man die Base so wählt, daß bei einer Temperatur zwischen 40 und 100°C gearbeitet wird.

10. Verfahren nach einem der Ansprüche 8 und 9, dadurch gekennzeichnet, daß die Reaktionszeit so gewählt wird, daß sie unter der zur Erzielung totaler Desacylierung des Ausgangs-Gangliosids notwendigen Zeit liegt.

11. Verfahren nach Anspruch 10, dadurch gekennzeichnet, daß die Reaktionszeit zwischen 30 min und 24 h liegt.

12. Verfahren nach einem der Ansprüche 8 bis 11, dadurch gekennzeichnet, daß die Base eine Base ist, die in Wasser einen pH von 9 bis 14 zu ergeben vermag.

13. Verfahren nach einem der Ansprüche 8 bis 12, dadurch gekennzeichnet, daß man mit einer Lösung einer Alkalinität arbeitet, die mit normaler Natron- oder Kalilauge bei 100°C nicht überschreitender Temperatur vergleichbar ist.

14. Verfahren nach Anspruch 13, dadurch gekennzeichnet, daß man bei etwa 60 bis 100°C etwa 2 h arbeitet.

15. Verfahren nach einem der Ansprüche 8 und 10 bis 12, dadurch gekennzeichnet, daß man mit einer Lösung einer Alkalinität vergleichbar einer zehntelnormalen Natronlauge bei einer Temperatur unter oder von 120°C arbeitet.

16. Verwendung von aktivierten Derivaten gemäß einem der Ansprüche 1 bis 7, dadurch gekennzeichnet, daß man einen Träger, auf den das aktivierte Derivat des Gangliosids fixiert ist, mit einer das Tetanustoxin, das man reinigen oder isolieren will, enthaltenden Lösung zusammen bringt.

17. Verwendung nach Anspruch 6, dadurch gekennzeichnet, daß man zum Abtrennen des Tetanustoxins von einer dieses enthaltenden oder möglicherweise enthaltenden Lösung eine Säule mit einem aktivierten Derivat des Gangliosids $G_{D1b}$ und/oder $G_{D1a}$ und/oder $G_{T1}$, auf einem Träger fixiert, herstellt, die Lösung über die Säule laufen läßt und, wenn gewünscht, dann das Toxin eluiert.

## Claims

1. Partially deacylated activated derivatives of gangliosides having a N-acyl group, characterised in that the said gangliosides are chosen from amongst the gangliosides $G_{D1a}$, $G_{D1b}$ and $G_{T1}$, that they exhibit free amine groups which can be detected by a positive reaction in the ninhydrin test, that they are mobile on thin-layer chromatography on silica gel in a chloroform/methanol/water (60:32:7) system and that they exhibit specific affinity characteristics toward tetanus toxin, it being possible for the said derivatives to be coupled to solid carriers via amine groups which have appeared in the course of the aprtial deacylation, without loss of the said specific affinity characteristics.

2. Derivatives according to Claim 1, characterised in that the ganglioside is ganglioside $G_{D1a}$.

3. Derivatives according to Claim 1, characterised in that the ganglioside is ganglioside $G_{D1b}$.

4. Derivatives according to Claim 1, characterised in that the ganglioside is ganglioside $G_{T1}$.

5. Derivatives according to any one of Claims 1 to 4, characterised in that the said partial deacylation products are immobilised on a solid organic or inorganic carrier.

6. Derivatives according to the preceding claim, characterised in that the said carrier is a modified or unmodified organic polymer, the said ganglioside derivative being fixed to the carrier via amine groups which have appeared in the course of the partial deacylation.

7. Derivatives according to Claim 5, characterised in that the said carrier is a porous inorganic body which has been modified to cause reactive groups to appear which are capable of reacting with the reactive groups of a coupling agent or with the $NH_2$ groups of the said deacylated derivatives.

8. Process for the preparation of the derivatives according to any one of the preceding claims, characterised in that one of the said gangliosides or a mixture of the said gangliosides is treated with a basic aqueous solution at a temperature of between 0 and 120°C, the temperature being the lower the more basic the medium.

9. Process according to Claim 8, characterised in that the base is chosen so that the process is carried out at a temperature of between 40 and 100°C.

10. Process according to any one of Claims 8 and 9, characterised in that the reaction time is so chosen as to be less than the time required to achieve complete deacylation of the starting ganglioside.

11. Process according to Claim 10, characterised in that the reaction time is between 30 minutes and 24 hours.

12. Process according to any one of Claims 8 to 11, characterised in that the base is a base capable of giving a pH of 9 to 14 in water.

13. Process according to any one of Claims 8 to 12, characterised in that it is carried out with a solution whose alkalinity is comparable to that of normal sodium hydroxide or potassium

hydroxide, at a temperature not exceeding 100°C.

14. Process according to Claim 13, characterised in that it is carried out at 60—100°C for 2 hours.

15. Process according to any one of Claims 8 and 10 to 12, characterised in that it is carried out with a solution of alkalinity comparable to that of decinormal sodium hydroxide, at a temperature below or equal to 120°C.

16. Application of the activated derivatives according to any one of Claims 1 to 7, characterised in that a carrier on which the said activated ganglioside derivative is immobilised is brought into contact with a solution containing or capable of containing the tetanus toxin which it is desired to purify or isolate.

17. Application according to Claim 16, characterised in that in order to separate the tetanus toxin from a solution containing or capable of containing it, a column is made up with an activated derivative of ganglioside $G_{D1b}$ and/or $G_{D1a}$ and/or $G_{T1}$ immobilised on a carrier, the said solution is passed over the said column and, if desired, the toxin is subsequently eluted.